(19) Europäisches Patentamt
European Patent Office
Office européen des brevets

(11) **EP 0 577 526 B1**

(12) **FASCICULE DE BREVET EUROPEEN**

(45) Date de publication et mention de la délivrance du brevet:
**20.11.2002 Bulletin 2002/47**

(51) Int Cl.[7]: **C08F 220/04**, C08F 220/28, C09D 7/00

(21) Numéro de dépôt: **93420239.1**

(22) Date de dépôt: **11.06.1993**

(54) **Copolymère acrylique partiellement ou totalement hydrosoluble, réticulé ou non et son utilisation**

Acrylat-Copolymer, teilweise oder ganz löslich in Wasser, vernetzt oder nicht und seine Verwendung

Acrylic copolymer partially or fully soluble in water, cured or not and its use

(84) Etats contractants désignés:
**BE CH DE DK ES GB IT LI NL SE**

(30) Priorité: **01.07.1992 FR 9208399**

(43) Date de publication de la demande:
**05.01.1994 Bulletin 1994/01**

(60) Demande divisionnaire:
**02076892.5**

(73) Titulaire: **COATEX S.A.S.**
**69730 Genay (FR)**

(72) Inventeurs:
- **Egraz, Jean-Bernard**
**F-69130 Ecully (FR)**
- **Grondin, Henri**
**F-69004 Lyon (FR)**
- **Suau, Jean-Marc**
**F-69009 Lyon (FR)**

(56) Documents cités:
**EP-A- 0 003 235**
**EP-A- 0 011 806**
**EP-A- 0 013 836**
**EP-A- 0 577 525**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 577 526 B1

## Description

La présente invention se rapporte à un copolymère partiellement ou totalement hydrosoluble réticulé ou non et constitué de motifs monomériques distincts composés d'au moins un monomère à insaturation éthylénique et à fonction carboxylique, éventuellement au moins un monomère à insaturation éthylénique et sans fonction carboxylique, au moins un monomère oxyalkylé à insaturation éthylénique et terminé par une chaîne grasse hydrophobe possédant au moins 26, et jusqu'à 30 inclus, atomes de carbone et éventuellement au moins un monomère possédant au moins deux insaturations éthyléniques.

La présente invention concerne également l'application de ce copolymère comme modificateur de rhéologie dans des applications aussi diverses que les boues de forage, les pâtes d'impression textile, la cosmétique, ou encore la détergence, et autres compositions de revêtement comme la peinture et comme agent antisédimentation et/ou de mise en suspension pour des charges minérales ou organiques grossières dans divers domaines comme par exemple le phytosanitaire.

On connait déjà des copolymères formés de motifs monomériques dont le premier est carboxylé et le deuxième non.

Ainsi, le brevet EP 0 173 109 décrit un copolymère constitué d'acide carboxylique à insaturation éthylénique, d'ester à insaturation éthylénique et d'un troisième monomère produit de la réaction d'un alcool gras contenant 6 à 22 atomes de carbone avec un isocyanate insaturé.

De même, le brevet EP 0 013 836 révèle, mais seulement de manière très générale, un copolymère à base d'acide méthacrylique, d'acrylate d'alkyle ayant jusqu'à 4 atomes de carbone et d'un monomère oxyalkylé terminé par une chaîne grasse ayant jusqu'à 30 atomes de carbone, tout comme le brevet EP 0 011 806 qui décrit également le même type de copolymère dont le troisième monomère est un monomère à insaturation éthylénique et terminé par une chaîne grasse possédant jusqu'à 20 atomes de carbone, ou encore le brevet EP 0 248 612 qui revendique un copolymère dont le troisième monomère est un ester dont la chaîne grasse contient jusqu'à 25 atomes de carbone.

On connait également un autre brevet EP 0 216 479 qui décrit, mais seulement de manière très générale, un copolymère à base de monomère ionique à insaturation éthylénique, de monomère substantiellement non ionique à insaturation éthylénique, d'un éther allylique oxyalkylé ayant jusqu'à 30 atomes de carbone et d'un réticulant.

Mais toute cette littérature décrit des polymères dont l'utilisation dans certaines compositions aqueuses ne donne pas entière satisfaction.

Le problème que la présente invention vise à résoudre consiste à augmenter l'épaississement de compositions aqueuses sous bas gradient de cisaillement grâce à l'efficacité épaississante accrue des polymères selon l'invention et à conférer aux suspensions aqueuses de matières minérales ou organiques des stabilités remarquables, notamment sur le plan de l'antisédimentation sans qu'il y ait pour autant d'augmentation notable des viscosités comme c'est souvent le cas des polymères décrits dans l'art antérieur.

Ces comportements très spécifiques des polymères de l'invention sont apportés par le choix et la nature des motifs monomériques constituants les macromolécules.
L'homme du métier saura envisager des produits équivalents aux produits décrits dans la présente demande notamment en faisant varier dans le troisième monomère le nombre de moles d'oxyde d'alkylène condensé, et la longueur de la chaîne grasse hydrophobe de façon à conserver des propriétés équivalentes aux produits exemplifiés. L'homme du métier pourra notamment préconiser le nombre d'oxyde d'alkylène en fonction du nombre de carbone de la chaîne grasse et en fonction de l'application.

Dans un souci de clarté dans toute la présente demande il ne sera fait référence qu'au nombre de carbone de la chaîne grasse hydrophobe du monomère spécial oxyalkylé.

La présente invention a donc d'abord pour objet un copolymère de formule générale :

$$(A)_a — (B)_b — (C)_c — (D)_d$$

dans laquelle :

(A) représente le ou les monomères à insaturation éthylénique et à fonction carboxylique avec "a" le pourcentage pondéral du monomère (A) par rapport au poids total des monomères,

(B) représente le ou les monomères à insaturation éthylénique et sans fonction carboxylique avec "b" le pourcentage pondéral du monomère (B) par rapport au poids total des monomères, "b" pouvant être nul.

(C) représente le monomère oxyalkylé à insaturation éthylénique et terminé par une chaîne grasse hydrophobe possédant au moins 26, et jusqu'à 30, atomes de carbone avec "c" le pourcentage pondéral du monomère (C)

par rapport au poids total des monomères.

Le monomère (C) sera nommé dans la suite de la description "monomère spécial" et s'écrit suivant la formule (I) suivante :

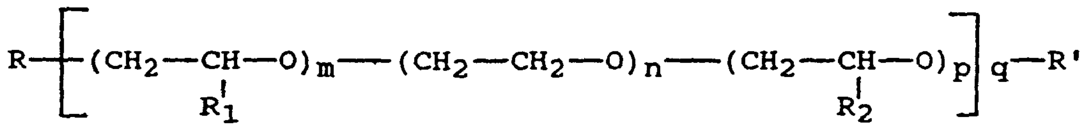

dans laquelle :

- m et p représentent un nombre de motifs d'oxyde d'alkylène,
- n représente un nombre de motifs d'oxyde d'éthylène,
- q un nombre au moins égal à 1 et tels que :

$$O < q(n + m + p) \leq 100,$$

- R le radical insaturé polymérisable,
- R' le radical hydrophobe à chaîne grasse possédant au moins 26, et jusqu'à 30 inclus, atomes de carbone,
- R1 l'hydrogène ou le groupe méthyle,
- R2 l'hydrogène ou le groupe méthyle.

(D) représente le ou les monomères possédant au moins deux insaturations éthyléniques avec "d" le pourcentage pondéral du monomère (D) par rapport au poids total des monomères, "d" pouvant être nul et tel que a + b + c + d = 100%.

Ainsi, alors que l'art antérieur décrit de très nombreux copolymères à motifs monomériques distincts donnant aux compositions aqueuses un comportement rhéologique aboutissant à des augmentations notables des viscosités, le copolymère selon l'invention s'en distingue par le fait qu'il est constitué :

a. d'au moins un monomère à insaturation éthylénique et à fonction carboxylique choisi parmi les monoacides, tels que l'acide acrylique, méthacrylique, crotonique, isocrotonique, cinnamique, les diacides, tels que l'acide itaconique, fumarique, maléïque, citraconique, les anhydrides d'acides carboxyliques, tels que l'anhydride maléïque et les hémiesters de diacides, tels que les monoesters en $C_1$ à $C_4$, des acides maléïque ou itaconique.
Toutefois, le monomère éthylénique carboxylé est préférentiellement choisi dans le groupe constitué par les acides acrylique, méthacrylique et itaconique,

b. éventuellement d'au moins un monomère à insaturation éthylénique et sans fonction carboxylique, choisi dans le groupe constitué par les esters d'acides acrylique ou méthacrylique, tels que les acrylates ou méthacrylates de méthyle, éthyle, butyle, 2-éthyl-hexyle, ou encore parmi l'acrylonitrile, l'acétate de vinyle, le styrène, le méthylstyrène, le diisobutylène, la vinylpyrolidone, la vinylcaprolactame.
Cependant, le monomère à insaturation éthylénique sans fonction carboxylique est préférentiellement choisi parmi les esters acryliques, tels que les acrylates et méthacrylates d'alkyle en $C_1$ à $C_4$.

c. d'au moins un monomère appelé "monomère spécial" oxyalkylé à insaturation éthylénique et terminé par une chaîne grasse hydrophobe, de formule générale (I) :

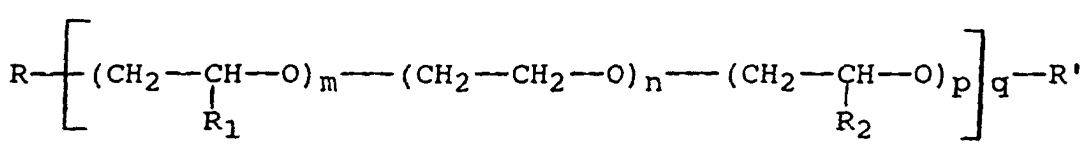

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$O < q(n + m + p) \leq 100,$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes choisis parmi les acryluréthanne, méthacryluréthanne, $\alpha$-$\alpha$-diméthyl-m-isopropenylbenzyluréthanne, allyluréthanne ou bien encore les éthers allyliques, les acrylamide et méthacrylamide substituées ou non, des vinyliques.

R' représente le radical hydrophobe à chaîne grasse constitué des groupes linéaires ou ramifiés alkyle, alkylaryle, arylalkyle, aryle ayant au moins 26 atomes de carbone et jusqu'à 30 atomes de carbone inclus. Cependant, il est également choisi de manière préférentielle un radical hydrophobe alkyle linéaire ou ramifié à 28 atomes de carbone et un nombre d'oxyde d'alkylène variant de 10 à 70.

d. éventuellement d'au moins un monomère possédant au moins deux insaturations éthyléniques choisi dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, les triallylcyanurates, les éthers allyliques obtenus à partir de polyols choisis parmi le pentaérythritol, le sorbitol, le sucrose et en ce que ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat™ 115 équipé d'un mobile 145 et pour un taux de cisaillement de 0,1 $s^{-1}$.

Le copolymère selon l'invention, obtenu par des procédés connus de copolymérisation radicalaire en solution, en émulsion directe ou inverse, en suspension ou précipitation dans des solvants appropriés, en présence de systèmes catalytiques et d'agents de transfert connus, contient préférentiellement, exprimé en pour cent en poids :

a. de 15% à 98%, et tout particulièrement de 20% à 50% de monomère(s) à insaturation éthylénique ayant au moins une fonction carboxylique,

b. de 0% à 83%, et tout particulièrement de 47% à 77% d'autre(s) monomère(s) à insaturation éthylénique démuni (s) de fonction carboxylique,

c. de 2% à 18% et tout particulièrement de 3% à 10% de monomère spécial.

d. de 0% à 5% et tout particulièrement de 0% à 3% de monomère(s) possédant au moins deux insaturations éthyléniques.

Le total des constituants (a), (b), (c) et (d) étant égal à 100.

L'invention concerne également les compositions aqueuses chargées et/ou pigmentées contenant le copolymère selon l'invention. Les compositions aqueuses chargées et/ou pigmentées sont plus particulièrement celles qui, outre le copolymère selon l'invention, contiennent une charge minérale telle que le carbonate de calcium, les argiles, les oxydes de fer, les silico-aluminates de sodium ou zéolithes et/ou un ou plusieurs colorants et éventuellement un liant naturel ou synthétique ainsi qu'éventuellement d'autres constituants comme des dispersants, des agents de coalescence, des biocides, des tensio-actifs, des antimousses.

Parmi toutes ces compositions aqueuses, contenant le copolymère selon l'invention, on peut par exemple citer les compositions cosmétiques, les pâtes d'impression textile, les suspensions aqueuses de zéolithes, les fluides de forage en particulier à l'eau, les formulations de crème à récurer, les formulations de détergence, les peintures et autres compositions de revêtement.

La portée et l'intérêt de l'invention seront mieux perçus grâce aux exemples suivants qui ne sauraient être limitatifs.

**Exemple 1 :**

Afin de comparer l'efficacité à l'épaississement sous bas gradient de cisaillement de différents épaississants, on prépare des solutions aqueuses de polymères à 0,5% en poids sec puis on les neutralise à pH = 8,5 à l'aide d'ammoniaque à 28% avant de les laisser au repos 24 heures.

Au bout de ces 24 heures les viscosités apparentes de ces gels sont déterminées à 25°C à l'aide du viscosimètre Rhéomat 115 commercialisé par la société CONTRAVES et équipé du mobile 145 et pour les taux de cisaillement 0,1 et 0,5 $s^{-1}$.

Dans tous les essais de l'exemple, les copolymères étudiés sont constitués de :

a. 36% en poids d'acide méthacrylique,
b. 55% en poids d'acrylate d'éthyle,
c. 9% en poids du monomère spécial de formule générale (I),

Ainsi, sont testés pour :

L'essai n°1 :

un copolymère selon l'art antérieur dont le monomère spécial de formule générale (I) est un hémimaléate comprenant 25 motifs d'oxyde d'éthylène et un radical R' alkyle linéaire constitué de 12 atomes de carbone.

L'essai n°2 :

Un copolymère selon l'art antérieur dont le monomère spécial de formule générale (I) est un hémimaléate comprenant 25 motifs d'oxyde d'éthylène et un radical R' alkyle linéaire constitué de 16 à 18 atomes de carbone.

L'essai n°3 :

Un copolymère selon l'art antérieur dont le monomère spécial de formula générale (I) est un hémimaléate comprenant 25 motifs d'oxyde d'éthylène et un radical R' alkyle constitué de 22 atomes de carbone.

L'essai n°4 :

Un copolymère selon l'invention dont le monomère spécial de formule générale (I) est un hémimaléate comprenant 25 motifs d'oxyde d'éthylène et un radical R' alkyle constitué de 28 atomes de carbone.

L'essai n°5 :

Un copolymère selon l'art antérieur dont le monomère spécial de formule générale (I) est un méthacrylate comprenant 25 motifs d'oxyde d'éthylène et un radical R' alkyle linéaire constitué de 12 atomes de carbone.

L'essai n°6 :

Un copolymère selon l'art antérieur dont le monomère spécial de formule générale (I) est un méthacrylate comprenant 25 motifs d'oxyde d'éthylène et un radical R' alkyle linéaire constitué de 16 à 18 atomes de carbone.

L'essai n°7 :

Un copolymère selon l'art antérieur dont le monomère spécial de formule générale (I) est un méthacrylate comprenant 25 motifs d'oxyde d'éthylène et un radical R' alkyle constitué de 22 atomes de carbone.

Les résultats des mesures en Pa.s des viscosités apparentes sous les différents taux de cisaillement en s-1 sont rassemblés pour tous les essais dans le tableau I suivant :

TABLEAU I

| | ESSAI N° | MONOMERE DE FORMULE GENERALE I | | | | | | | | TAUX DE CISAILLEMENT EN S $^{-1}$ | |
|---|---|---|---|---|---|---|---|---|---|---|---|
| | | m | n | p | q | $R_1$ | $R_2$ | R | R' | 0,1 | 0,5 |
| | | | | | | | | | | η en Pa.s | η en Pa.s |
| A.A. | 1 | 0 | 25 | 0 | 1 | - | - | hémimaléate | alkyle en C 12 | 5,75 | 1,15 |
| A.A. | 2 | 0 | 25 | 0 | 1 | - | - | hémimaléate | céto-stéaryle | 6,23 | 1,25 |
| A.A. | 3 | 0 | 25 | 0 | 1 | - | - | hémimaléate | alkyle en C 22 | 5,27 | 1,92 |
| INV. | 4 | 0 | 25 | 0 | 1 | - | - | hémimaléate | alkyle en C 28 | 52 | 26 |
| A.A. | 5 | 0 | 25 | 0 | 1 | - | - | méthacrylate | alkyle en C 12 | 0,48 | 0,19 |
| A.A. | 6 | 0 | 25 | 0 | 1 | - | - | méthacrylate | céto-stéaryle | 42,2 | 14,5 |
| A.A. | 7 | 0 | 25 | 0 | 1 | - | - | méthacrylate | alkyle en C 22 | 40 | 12,3 |

η = viscosité apparente

A.A. = ART ANTERIEUR
INV. = INVENTION

La lecture du tableau I permet de constater que pour l'essai n°4, l'épaississement du gel à 0,5% en poids sec de polymère est nettement supérieur pour les copolymères selon l'invention constitués d'acide méthacrylique, d'acrylate d'éthyle et d'un monomère oxyalkylé à insaturation éthylénique et terminé par une chaîne grasse hydrophobe, de formule générale (I) dans laquelle R' représente le radical hydrophobe ayant au moins 26, et jusqu'à 30 inclus, atomes de carbone.

**Exemple 2 :**

Cet exemple concerne l'épaississement d'une base de shampooing contenant du lauryl éther sulfate d'ammonium et des silicones.

Mode opératoire :

La base à épaissir est introduite dans un flacon en verre et mise en agitation très lente à l'aide d'un système d'agitation du type Rayneri.
On pèse successivement et on introduit :

- la base du shampooing à épaissir : 250 g
- l'eau de qualité industrielle : 25 g
- puis l'épaississant à tester (contenant 30% en masse de matières actives) : 5 g

Le pH est réglé à 6,5 à l'aide de triéthanolamine.
Il est nécessaire d'obtenir un mélange en fin de formulation qui soit convenablement épaissi et qui soit également parfaitement limpide et transparent.
La mesure de la viscosité apparente est effectuée à l'aide d'un viscosimètre Brookfield du type RVT équipé du module adapté à 20°C.
Les lectures sont effectuées à 10 tours/minute et 100 tours/minute immédiatement après la réalisation du mélange et 48 heures après.
Les différents copolymères épaississants testés sont pour :

L'essai n°8

un copolymère selon l'art antérieur constitué de :

a. 36% en poids d'acide méthacrylique,
b. 55% en poids d'acrylate d'éthyle,
c. 9% en poids d'un méthacrylate comprenant 50 motifs d'oxyde d'éthylène et un radical R' alkyle linéaire constitué de 16 à 18 atomes de carbone.

L'essai n°9

un copolymère selon l'art antérieur constitué de :

a. 36% en poids d'acide méthacrylique,
b. 55% en poids d'acrylate d'éthyle,
c. 9% en poids d'un méthacrylate comprenant 20 motifs d'oxyde d'éthylène et un radical R' alkyle constitué de 22 atomes de carbone.

L'essai n°10

un copolymère selon l'art antérieur constitué de :

a. 36% en poids d'acide méthacrylique,
b. 55% en poids d'acrylate d'éthyle,
c. 9% en poids d'un méthacrylate comprenant 50 motifs d'oxyde d'éthylène et un radical R' alkylaryle nonylphényle.

L'essai n°11

un copolymère selon l'art antérieur constitué de :

a. 36% en poids d'acide méthacrylique,
b. 55% en poids d'acrylate d'éthyle,
c. 9% en poids d'un méthacrylate comprenant 35 motifs d'oxyde d'éthylène et un radical R' alkyle constitué de 22 atomes de carbone.

Les résultats des mesures des viscosités Brookfield des différents essais sont rassemblés dans le tableau II suivant.

TABLEAU II

| | ESSAI N° | MONOMERE DE FORMULE GENERALE I: m | n | p | q | $R_1$ | $R_2$ | R' | η IMMEDIATE EN mPa.s: 10 t/mn | 100 t/mn | η 48 HEURES EN mPa.s: 10 t/mn | 100 t/mn | ASPECT DU GEL |
|---|---|---|---|---|---|---|---|---|---|---|---|---|---|
| A.A. | 8 | 0 | 50 | 0 | 1 | - | - | céto-stéaryle | 600 | 560 | 1300 | 1050 | trouble |
| A.A. | 9 | 0 | 20 | 0 | 1 | - | - | alkyle en C22 | 400 | 420 | 500 | 500 | opaque |
| A.A. | 10 | 0 | 50 | 0 | 1 | - | - | nonyl-phenyle | 500 | 520 | 700 | 590 | trouble |
| A.A. | 11 | 0 | 35 | 0 | 1 | - | - | alkyle en C22 | 600 | 590 | 1000 | 800 | trouble |

η = viscosité Brookfield

A.A. = ART ANTERIEUR

INV. = INVENTION

Cette base de shampooing est considérée comme bonne si sa viscosité Brookfield à 10 tours/minute et 20°C est supérieure à 1000 mPa.s après 48 heures et si sa formulation est limpide.

La lecture du tableau II nous permet de distinguer que les essais de l'art antérieur ne constituent pas une bonne base de shampooing.

**Exemple 6 :**

Cet exemple concerne l'épaississement d'une base de crème à récurer contenant un mélange de tensio-actif non ionique et du carbonate de calcium de diamètre moyen égal à 30µm commercialisé par la société OMYA sous le nom de Durcal 40.

Mode opératoire :

Dans un flacon en verre de 1000 ml équipé d'un système d'agitation du type Rayneri, on introduit successivement et sous agitation :

- 330 g d'eau brute,
- 2,25 g d'un dispersant acrylique commercialisé par la société COATEX sous le nom de COATEX P90,
- et 375 g du carbonate de calcium Durcal 40.

La suspension aqueuse de carbonate de calcium ainsi obtenue est alors alcalinisée jusqu'à pH = 9,5 à l'aide d'ammoniaque à 28%.
Une fois le pH atteint on incopore, toujours sous agitation, 10 g du copolymère à tester contenant 30% de matière active et 3 g du tensio-actif non ionique du type nonylphénol condensé avec 10 molécules d'oxyde d'éthylène.
Après 5 minutes d'agitation, on stoppe celle-ci et on estime la sédimentation immédiate ($T_o$) à l'aide d'une spatule introduite dans la base de crème à récurer. On peut alors distinguer la sédimentation particulièrement rapide par le fait que la spatule rencontre une résistance à la pénétration au fond du flacon.
Les bases de crème à récurer sont alors laissées au repos pendant 48 heures.
Au bout de ces 48 heures, on mesure d'une part la viscosité apparente Brookfield à 20°C et à 10 tours/minute et 100 tours/minute au moyen d'un viscosimètre Brookfield type RVT équipé du module adéquat et d'autre part la sédimentation (T48H) du mélange estimé de la même façon que la sédimentation immédiate.
Les différents copolymères testés sont pour :

L'essai n°12 :

un copolymère selon l'art antérieur constitué de :

a. 36% en poids d'acide méthacrylique,
b. 55% en poids d'acrylate d'éthyle
c. 9% en poids d'un hémimaléate comprenant 25 motifs d'oxyde d'éthylène et un radical R' alkyle linéaire constitué de 16 à 18 atomes de carbone.

L'essai n°13 :

un copolymère selon l'art antérieur constitué de :

a. 36% en poids d'acide méthacrylique,
b. 55% en poids d'acrylate d'éthyle,
c. 9% en poids d'un méthacrylate comprenant 25 motifs d'oxyde d'éthylène et un radical R' alkyle linéaire constitué de 16 à 18 atomes de carbone.

L'essai n°14 :

un copolymère selon l'art antérieur constitué de :

a. 36% en poids d'acide méthacrylique,
b. 55% en poids d'acrylate d'éthyle,
c. 9% en poids d'un hémimaléate comprenant 25 motifs d'oxyde d'éthylène et un radical R'alkyle constitué de 22 atomes de carbone.

L'essai n°15 :

un copolymère selon l'art antérieur constitué de :

a. 36% en poids d'acide méthacrylique,
b. 55% en poids d'acrylate d'éthyle,
c. 9% en poids d'un méthacrylate comprenant 25 motifs d'oxyde d'éthylène et un radical R' alkyle constitué de 22 atomes de carbone.

Les résultats des mesures de viscosité apparente Brookfield à 10 tours/minute et 100 tours/minute à 20°C après 48 heures de repos ainsi que ceux de sédimentation (To) et de sédimentation (T48H) après 48 heures de repos des différents essais sont rassemblés dans le tableau VI suivant.

TABLEAU VI

| | ESSAI N° | MONOMERE DE FORMULE GENERALE I | | | | | | | | SEDIMENTATION | η 48H EN mPa.s | | SEDIMENTATION |
|---|---|---|---|---|---|---|---|---|---|---|---|---|---|
| | | m | n | p | q | $R_1$ | $R_2$ | R | R' | $T_0$ | 10 t/mn | 100 t/mn | $T_{48H}$ |
| A.A. | 12 | 0 | 25 | 0 | 1 | — | — | hémimaléate | céto-stéaryle | OUI | < 300 | — | OUI |
| A.A. | 13 | 0 | 25 | 0 | 1 | — | — | méthacrylate | céto-stéaryle | OUI | < 300 | — | OUI |
| A.A. | 14 | 0 | 25 | 0 | 1 | — | — | hémimaléate | alkyle à 22 C | OUI | < 300 | — | OUI |
| A.A. | 15 | 0 | 25 | 0 | 1 | — | — | méthacrylate | alkyle à 22 C | OUI | < 300 | — | OUI |

η = viscosité Brookfield

A.A. = ART ANTERIEUR
INV. = INVENTION

Une base de crème à récurer est considérée comme bonne si, après 48 heures de stockage, le système épaissi en présence de tensio-actif non ionique présente une viscosité apparente Brookfield supérieure à 3000 mPa. s à 10 tours/minute et aucune sédimentation au bout d'au moins 48 heures.

La lecture du tableau VI permet de constater que les essais de l'art antérieur ne donnent pas de formulations à viscosité apparente élevée et sans sédimentation.

## Revendications

### Revendications pour les Etats contractants suivants : BE, CH, DE, DK, GB, IT, LI, NL, SE

**1.** Copolymère partiellement ou totalement hydrosoluble, réticulé ou non, **caractérisé en ce qu'**il est constitué :

a. d'au moins un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides tels que les monoesters en $C_1$ à $C_4$, des acides maléique ou itaconique,

b. éventuellement d'au moins un monomère à insaturation éthylénique et sans fonction carboxylique, choisi dans le groupe constitué par les esters, les amides et/ou les nitriles des acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. d'au moins un monomère oxyalkylé à insaturation éthylénique et terminé par une chaîne grasse hydrophobe, de formule générale (I) :

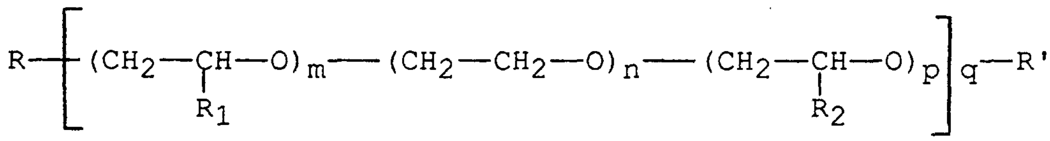

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$O < q(n+m+p) \leq 100$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes choisis parmi les acryluréthanne, méthacryluréthanne, α-α diméthyl-m-isopropenylbenzyluréthanne, allyluréthanne ou bien encore les éthers allyliques, les acrylamide et méthacrylamide substituées ou non, des vinyliques.

R' représente le radical hydrophobe à chaîne grasse constitué des groupes linéaires ou ramifiés alkyle, alkylaryle, arylalkyle, aryle ayant de 26 atomes de carbone à 30 atomes de carbone inclus.

d. éventuellement d'au moins un monomère possédant au moins deux insaturations éthyléniques choisi dans le groupe constitué par le diméthacrylate d'éthylèneglycol, le triméthylolpropanetriacrylate, l'acrylate d'allyle, le méthylène-bis-acrylamide, le méthylène-bis-méthacrylamide, le tétrallyloxyéthane, le triallylcyanurate, les

éthers allyliques obtenus à partir de polyols choisis parmi le pentaérythritol, le sorbitol, le sucrose

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**2.** Copolymère partiellement ou totalement hydrosoluble selon la revendication 1, **caractérisé en ce qu'**il est constitué :

a. d'au moins un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides tels que les monoesters en $C_1$ à $C_4$, des acides maléique ou itaconique,

b. éventuellement d'au moins un monomère à insaturation éthylénique et sans fonction carboxylique, choisi dans le groupe constitué par les esters, les amides et/ou les nitriles des acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. d'au moins un monomère oxyalkylé à insaturation éthylénique et terminé par une chaîne grasse hydrophobe, de formule générale (I) :

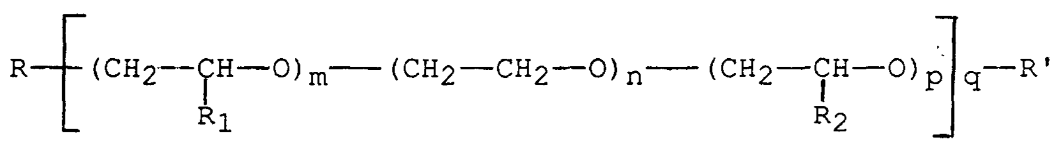

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$O < q<n+m+p) \leq 100$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes choisis parmi les acryluréthanne, méthacryluréthanne, $\alpha$-$\alpha$ diméthyl-m-isopropenylbenzyluréthanne, allyluréthanne ou bien encore les éthers allyliques, les acrylamide et méthacrylamide substituées ou non, des vinyliques.
R' représente le radical hydrophobe à chaîne grasse constitué des groupes linéaires ou ramifiés alkyle ayant 28 atomes de carbone.

d. éventuellement d'au moins un monomère possédant au moins deux insaturations éthyléniques choisi dans le groupe constitué par le diméthacrylate d'éthylèneglycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**3.** Copolymère partiellement ou totalement hydrosoluble selon les revendications 1 ou 2 **caractérisé en ce qu'**il est constitué :

a. de 15% à 98% en poids d'un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides choisis parmi les monoesters en $C_1$ à $C_4$, des acides maléique ou itaconique,

b. de 0% à 83% en poids d'un monomère à insaturation éthylénique et sans fonction carboxylique, choisi dans le groupe constitué par les esters d'acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. de 2% à 18% en poids d'un monomère oxyalkylé à insaturation éthylénique et terminé par une chaîne grasse hydrophobe, de formule générale (I) selon les revendications 1 ou 2.

d. de 0% à 5% d'au moins un monomère possédant au moins deux insaturations éthyléniques choisi dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**4.** Copolymère selon les revendications 1 ou 2 **caractérisé en ce qu'**il est constitué :

a. de 20% à 50% en poids d'un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides choisis parmi les monoesters en $C_1$ à $C_4$, des acides maléique ou itaconique,

b. de 47% à 77% en poids d'un monomère à insaturation éthylénique et sans fonction carboxylique, choisi dans le groupe constitué par les esters d'acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. de 3% à 10% en poids d'un monomère oxyalkylé à insaturation éthylénique et terminé par une chaîne grasse hydrophobe, de formule générale (I) selon les revendications 1 ou 2.

d. de 0% à 3% d'au moins un monomère possédant au moins deux insaturations éthyléniques choisi dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'une mobile 145 et pour un taux de cisaillement de 0,1 $^{s-1}$.

**5.** Copolymère selon l'une des revendications 1 à 4 **caractérisé en ce que** le monomère à insaturation éthylénique et à fonction carboxylique est choisi dans le groupe des acides acrylique, méthacrylique et itaconique.

**6.** Copolymère selon l'une des revendications 1 à 4 **caractérisé en ce que** le monomère à insaturation éthylénique sans fonction carboxylique est choisi parmi les esters acryliques tels que les acrylates et méthacrylates d'alkyle possédant de un à quatre atomes de carbone.

**7.** Utilisation du copolymère selon l'une des revendications 1 à 6 comme modificateur de rhéologie dans des applications cosmétiques.

**8.** Utilisation selon la revendication 7 dans des formulations du type shampooing.

**9.** Utilisation du copolymère selon l'une des revendications 1 à 6 comme modificateur de rhéologie dans des formulations pâte d'impression textile.

**10.** Utilisation du copolymère selon l'une des revendications 1 à 6 comme additif des boues de forage à l'eau.

**11.** Utilisation du copolymère selon l'une des revendications 1 à 6 comme modificateur de rhéologie dans des applications détergentes.

**12.** Utilisation selon la revendication 11 dans des formulations de type crème à récurer.

**13.** Utilisation du copolymère selon l'une des revendications 1 à 6 comme agent antisédimentation et/ou de mise en suspension de charges minérales ou organiques grossières pour diverses applications du type phytosanitaire.

**14.** Utilisation du copolymère selon l'une des revendications 1 à 6 comme modificateur de rhéologie dans les formulations peinture.

**Revendications pour l'Etat contractant suivant : ES**

**1.** Copolymère partiellement ou totalement hydrosoluble, réticulé ou non, **caractérisé en ce qu'**il est constitué :

a. d'au moins un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides tels que les monoesters en $C_1$ à $C_4$, des acides maléique ou itaconique,

b. éventuellement d'au moins un monomère à insaturation éthylénique et sans fonction carboxylique, choisi dans le groupe constitué par les esters, les amides et/ou les nitriles des acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. d'au moins un monomère oxyalkylé à insaturation éthylénique et terminé par une chaîne grasse hydrophobe, de formule générale (I) :

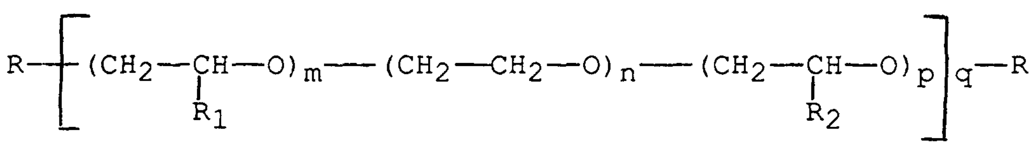

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$O < q(n+m+p) \leq 100$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes choisis parmi les acryluréthanne, méthacryluréthanne, α-α diméthyl-m-isopropenylbenzyluréthanne, allyluréthanne ou bien encore les éthers allyliques, les acrylamide et méthacrylamide substituées ou non, des vinyliques.

R' représente le radical hydrophobe à chaîne grasse constitué des groupes linéaires ou ramifiés alkyle, alkylaryle, arylalkyle, aryle ayant de 26 atomes de carbone à 30 atomes de carbone inclus.

d. éventuellement d'au moins un monomère possédant au moins deux insaturations éthyléniques choisi dans le groupe constitué par le diméthacrylate d'éthylèneglycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**2.** Copolymère partiellement ou totalement hydrosoluble selon la revendication 1, **caractérisé en ce qu'**il est constitué :

a. d'au moins un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, les anhydrides d'acides carboxyliques tels que l'anhydride maléique et les hémiesters de diacides tels que les monoesters en $C_1$ à $C_4$, des acides maléique ou itaconique,

b. éventuellement d'au moins un monomère à insaturation éthylénique et sans fonction carboxylique, choisi dans le groupe constitué par les esters, les amides et/ou les nitriles des acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. d'au moins un monomère oxyalkylé à insaturation éthylénique et terminé par une chaîne grasse hydrophobe, de formule générale (I) :

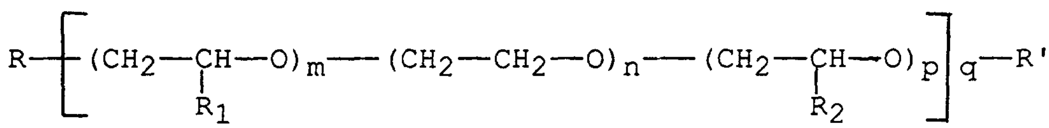

dans laquelle :

- m et p représentent un nombre de motifs d'oxyde d'alkylène inférieur ou égal à 100,
- n représente un nombre de motifs d'oxyde d'éthylène inférieur ou égal à 100,
- q un nombre au moins égal à 1 et tels que :

$$O < q(n+m+p) \leq 100$$

- $R_1$ l'hydrogène ou le radical méthyle,
- $R_2$ l'hydrogène ou le radical méthyle.

R représente le radical insaturé polymérisable, appartenant au groupe des esters acrylique, méthacrylique, maléique, itaconique, crotonique, vinylphtalique ainsi que les hémiesters maléique, itaconique, vinylphtalique ou encore les insaturés uréthannes choisis parmi les acryluréthanne, méthacryluréthanne, α-α diméthyl-m-isopropenylbenzyluréthanne, allyluréthanne ou bien encore les éthers allyliques, les acrylamide et méthacrylamide substituées ou non, des vinyliques.
R' représente le radical hydrophobe à chaîne grasse constitué des groupes linéaires ou ramifiés alkyle

ayant 28 atomes de carbone.

d. éventuellement d'au moins un monomère possédant au moins deux insaturations éthyléniques choisi dans le groupe constitué par le diméthacrylate d'éthylèneglycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**3.** Copolymère partiellement ou totalement hydrosoluble selon les revendications 1 ou 2 **caractérisé en ce qu'**il est constitué :

a. de 15% à 98% en poids d'un monomère à insaturation éthylénique et à fonction carboxylique choisi parmi les monoacides tels que l'acide acrylique, méthacrylique, crotonique, isocrotonique, cinnamique, ou encore parmi l'acide itaconique, fumarique, maléique, citraconique, les anhydrides d'acides carboxyliques tels que l'anhydride maléique et les hémiesters de diacides choisis parmi les monoesters en $C_1$ à $C_4$, des acides maléique ou itaconique,

b. de 0% à 83% en poids d'un monomère à insaturation éthylénique et sans fonction carboxylique, choisi dans le groupe constitué par les esters d'acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. de 2% à 18% en poids d'un monomère oxyalkylé à insaturation éthylénique et terminé par une chaîne grasse hydrophobe, de formule générale (I) selon les revendications 1 ou 2.

d. de 0% à 5% d'au moins un monomère possédant au moins deux insaturations éthyléniques choisi dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5 par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $^{s-1}$.

**4.** Copolymère selon les revendications 1 ou 2 **caractérisé en ce qu'**il est constitué :

a. de 20% à 50% en poids d'un monomère à insaturation éthylénique et à fonction carboxylique choisi parmi les monoacides tels que l'acide acrylique, méthacrylique, crotonique, isocrotonique, cinnamique, les diacides tels que l'acide itaconique, fumarique, maléique, citraconique, ou parmi l'anhydride maléique et les hémiesters de diacides choisis parmi les monoesters en $C_1$ à $C_4$, des acides maléique ou itaconique,

b. de 47% à 77% en poids d'un monomère à insaturation éthylénique et sans fonction carboxylique, choisi dans le groupe constitué par les esters d'acides acrylique ou méthacrylique, tels que les acrylates ou méthacrylates de méthyle, éthyle, butyle, 2-éthyl-hexyle, ou par l'acrylonitrile, l'acétate de vinyle, le styrène, le méthylstyrène, le diisobutylène, la vinylpyrrolidone, la vinylcaprolactame,

c. de 3% à 10% en poids d'un monomère oxyalkylé à insaturation éthylénique et terminé par une chaîne grasse hydrophobe, de formule générale (I) selon les revendications 1 ou 2.

d. de 0% à 3% d'au moins un monomère possédant au moins deux insaturations éthyléniques choisi dans le groupe constitué par le diméthacrylate d'éthylène glycol, le triméthylolpropanetriacrylate, l'acrylate d'allyle, le méthylène-bis-acrylamide, le méthylène-bis-méthacrylamide, le tétrallyloxyéthane, le triallylcyanurate, les éthers allyliques obtenus à partir de polyols choisis parmi le pentaérythritol, le sorbitol, le sucrose,

et **en ce que** ledit copolymère, mis en solution aqueuse à 0,5 % en poids de matière sèche portée à un pH de 8,5

par addition d'ammoniaque à 28 %, a une viscosité apparente d'au moins 52 Pa.s mesurée à 25°C et après 24 heures à l'aide d'un viscosimètre Rhéomat 115 équipé d'un mobile 145 et pour un taux de cisaillement de 0,1 $s^{-1}$.

**5.** Copolymère selon l'une des revendications 1 à 4 **caractérisé en ce que** le monomère à insaturation éthylénique et à fonction carboxylique est choisi dans le groupe des acides acrylique, méthacrylique et itaconique.

**6.** Copolymère selon l'une des revendications 1 à 4 **caractérisé en ce que** le monomère à insaturation éthylénique sans fonction carboxylique est choisi parmi les esters acryliques tels que les acrylates et méthacrylates d'alkyle possédant de un à quatre atomes de carbone.

**7.** Un procédé pour l'obtention d'un copolymère partiellement ou totalement hydrosoluble, réticulé ou non, selon l'une des revendications 1 à 6, qui comprend la copolymérisation radicalaire des monomères à copolymériser, en solution, en émulsion directe ou inverse, en suspension ou précipitation dans des solvants appropriés, en présence de systèmes catalytiques et d'agents de transfert appropriés.

**8.** Procédé d'utilisation du copolymère selon l'une des revendications 1 à 6 comme modificateur de rhéologie dans des applications cosmétiques.

**9.** Procédé d'utilisation selon la revendication 8 dans des formulations du type shampooing.

**10.** Procédé d'utilisation du copolymère selon l'une des revendications 1 à 6 comme modificateur de rhéologie dans des formulations pâte d'impression textile.

**11.** Procédé d'utilisation du copolymère selon l'une des revendications 1 à 6 comme additif des boues de forage à l'eau.

**12.** Procédé d'utilisation du copolymère selon l'une des revendications 1 à 6 comme modificateur de rhéologie dans des applications détergentes.

**13.** Procédé d'utilisation selon la revendication 12 dans des formulations de type crème à récurer.

**14.** Procédé d'utilisation du copolymère selon l'une des revendications 1 à 6 comme agent antisédimentation et/ou de mise en suspension de charges minérales ou organiques grossières pour diverses applications du type phytosanitaire.

**15.** Procédé d'utilisation du copolymère selon l'une des revendications 1 à 6 comme modificateur de rhéologie dans les formulations peinture.

## Claims

### Claims for the following Contracting States : BE, CH, DE, DK, GB, IT, LI, NL, SE

**1.** Partially or totally hydrosoluble copolymer, cross-linked or not, **characterised by** the fact that it is formed:

a. of at least one ethylenically unsaturated monomer with a carboxyl function selected from the monoacids such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, such as $C_1$ to $C_4$ monoesters of maleic or itaconic acid,

b. optionally of at least one ethylenically unsaturated monomer with no carboxyl function, selected from the group formed by the esters, amides and/or nitriles of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

c. of at least one ethylenically unsaturated oxyalkylated monomer terminated by a hydrophobic fatty chain, of general formula (I):

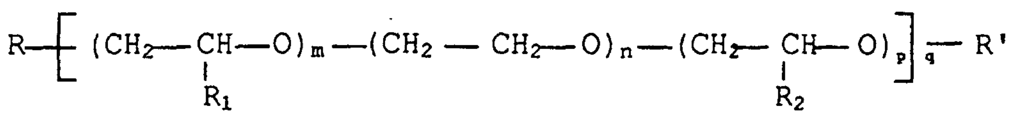

in which:

- m and p represent a number of alkylene oxide structures less than or equal to 100,
- n represents a number of ethylene oxide structures less than or equal to 100,
- q a number at least equal to 1 and such that:

$$0<q(n+m+p)\leq 100$$

- $R_1$ hydrogen or the methyl radical,
- $R_2$ hydrogen or the methyl radical,

R represents the polymerisable unsaturated radical belonging to the group of acrylic, methacrylic, maleic, itaconic, crotonic and vinylphthalic esters and maleic, itaconic and vinylphthalic hemiesters, or unsaturated urethanes selected from acrylic urethane, methacrylic urethane, α-α- dimethyl-m-isopropenylbenzyl urethane, allyl urethane, or allyl ethers, substituted or non-substituted vinyl acrylamide and methacrylamide,

R' represents the hydrophobic radical with a fatty chain formed of linear or branched alkyl, alkylaryl, arylalkyl, or aryl groups containing from 26 carbon atoms to 30 inclusive carbon atoms,

d. optionally, of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of 0.1 $s^{-1}$.

**2.** Partially or totally hydrosoluble copolymer according to the claim 1, **characterised by** the fact that it is formed:

a. of at least one ethylenically unsaturated monomer with a carboxyl function selected from the monoacids such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, such as $C_1$ to $C_4$ monoesters of maleic or itaconic acid,

b. optionally of at least one ethylenically unsaturated monomer with no carboxyl function, selected from the group formed by the esters, amides and/or nitriles of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

c. of at least one ethylenically unsaturated oxyalkylated monomer terminated by a hydrophobic fatty chain, of general formula (I):

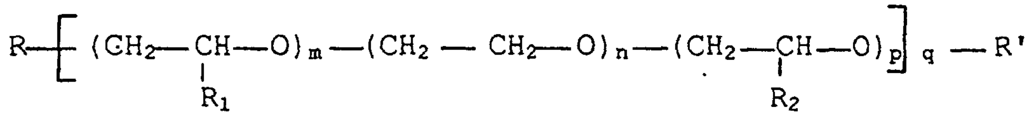

in which:

- m and p represent a number of alkylene oxide structures less than or equal to 100,
- n represents a number of ethylene oxide structures less than or equal to 100,
- q a number at least equal to 1 and such that:

$$0<q(n+m+p)\leq 100$$

- $R_1$ hydrogen or the methyl radical,
- $R_2$ hydrogen or the methyl radical,

R represents the polymerisable unsaturated radical belonging to the group of acrylic, methacrylic, maleic, itaconic, crotonic and vinylphthalic esters and maleic, itaconic and vinylphthalic hemiesters, or unsaturated urethanes selected from acrylic urethane, methacrylic urethane, α-α- dimethyl-m-isopropenylbenzyl urethane, allyl urethane, or allyl ethers, substituted or non-substituted vinyl acrylamide and methacrylamide,

R' represents the hydrophobic radical with a fatty chain formed of linear or branched alkyl groups having 28 carbon atoms,

d. optionally, of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of $0.1s^{-1}$.

**3.** Partially or totally hydrosoluble copolymer according to the claims 1 or 2, **characterised by** the fact that it is formed:

a. of 15% to 98% by weight of an ethylenically unsaturated monomer with a carboxyl function selected from the monoacids, such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, selected from $C_1$ to $C_4$ monoesters of maleic or itaconic acid,

b. of 0% to 83% by weight of an ethylenically unsaturated monomer with no carboxyl function, selected from the group formed by the esters of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

c. of 2% to 18% by weight of an ethylenically unsaturated oxyalkylated monomer terminated by a hydrophobic fatty chain, of general formula (I) according to the claims 1 or 2,

d. of 0% to 5% of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of $0.1\ s^{-1}$.

**4.** Copolymer according to the claims 1 or 2, **characterised by** the fact that it is formed:

a. of 20% to 50% by weight of an ethylenically unsaturated monomer with a carboxyl function selected from the monoacids, such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, selected from $C_1$ to $C_4$ monoesters of maleic or itaconic acid,

b. of 47% to 77% by weight of an ethylenically unsaturated monomer with no carboxyl function, selected from the group formed by the esters of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

c. from 3% to 10% by weight of an ethylenically unsaturated oxyalkylated monomer terminated by a hydrophobic fatty chain, of general formula (I) according to the claims 1 or 2,

d. of 0% to 3% of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of $0.1s^{-1}$.

**5.** Copolymer according to one of claims 1 to 4, **characterised by** the fact that the ethylenically unsaturated monomer with a carboxyl function is selected from the group of acrylic, methacrylic and itaconic acids.

**6.** Copolymer according to one of claims 1 to 4, **characterised by** the fact that the ethylenically unsaturated monomer with no carboxyl function is selected from acrylic esters such as alkyl acrylates and methacrylates having from one to four carbon atoms.

**7.** Use of the copolymer according to one of claims 1 to 6 as a rheology modifier in cosmetic applications.

**8.** Use according to the claim 7 in formulations of the shampoo type.

**9.** Use of the copolymer according to one of claims 1 to 6 as a rheology modifier in paste formulations for textile printing.

**10.** Use of the copolymer according to one of claims 1 to 6 as an additive to aqueous drilling muds.

**11.** Use of the copolymer according to one of claims 1 to 6 as a rheology modifier in detergent applications.

**12.** Use according to claim 11 in formulations of the scouring cream type.

**13.** Use of the copolymer according to one of claims 1 to 6 as an agent for preventing sedimentation and/or placing in suspension of coarse mineral or organic charges for various applications of the phytosanitary type.

**14.** Use of the copolymer according to one of claims 1 to 6 as a rheology modifier in paint formulations.

**Claims for the following Contracting State : ES**

**1.** Partially or totally hydrosoluble copolymer, cross-linked or not, **characterised by** the fact that it is formed:

**a.** of at least one ethylenically unsaturated monomer with a carboxyl function selected from the monoacids such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, such as $C_1$ to $C_4$ monoesters of maleic or itaconic acid,

**b.** optionally of at least one ethylenically unsaturated monomer with no carboxyl function, selected from the

group formed by the esters, amides and/or nitriles of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

**c.** of at least one ethylenically unsaturated oxyalkylated monomer terminated by a hydrophobic fatty chain, of general formula (I):

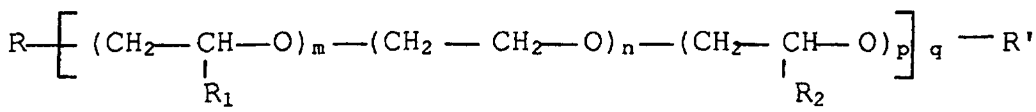

in which:

- m and p represent a number of alkylene oxide structures less than or equal to 100,
- n represents a number of ethylene oxide structures less than or equal to 100,
- q a number at least equal to 1 and such that:

$$0<q(n+m+p) \leq 100$$

- $R_1$ hydrogen or the methyl radical,
- $R_2$ hydrogen or the methyl radical,

R represents the polymerisable unsaturated radical belonging to the group of acrylic, methacrylic, maleic, itaconic, crotonic and vinylphthalic esters and maleic, itaconic and vinylphthalic hemiesters, or unsaturated urethanes selected from acrylic urethane, methacrylic urethane, α-α- dimethyl-m-isopropenylbenzyl urethane, allyl urethane, or allyl ethers, substituted or non-substituted vinyl acrylamide and methacrylamide,

R' represents the hydrophobic radical with a fatty chain formed of linear or branched alkyl, alkylaryl, arylalkyl, or aryl groups containing from 26 carbon atoms to 30 inclusive carbon atoms,

**d.** optionally, of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of 0.1 $s^{-1}$.

**2.** Partially or totally hydrosoluble copolymer according to the claim 1, **characterised by** the fact that it is formed:

**a.** of at least one ethylenically unsaturated monomer with a carboxyl function selected from the monoacids such as acrylic, methacrylic, crotonic, isocrotonic and cinnamic acid, the diacids such as itaconic, fumaric, maleic and citraconic acid, carboxylic acid anhydrides, such as maleic anhydride and diacid hemiesters, such as $C_1$ to $C_4$ monoesters of maleic or itaconic acid,

**b.** optionally of at least one ethylenically unsaturated monomer with no carboxyl function, selected from the group formed by the esters, amides and/or nitriles of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

**c.** of at least one ethylenically unsaturated oxyalkylated monomer terminated by a hydrophobic fatty chain, of general formula (I):

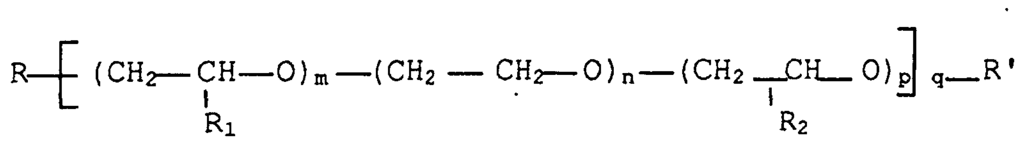

in which:

- m and p represent a number of alkylene oxide structures less than or equal to 100,
- n represents a number of ethylene oxide structures less than or equal to 100,
- q a number at least equal to 1 and such that:

$$0<q(n+m+p)\leq 100$$

- $R_1$ hydrogen or the methyl radical,
- $R_2$ hydrogen or the methyl radical,

R represents the polymerisable unsaturated radical belonging to the group of acrylic, methacrylic, maleic, itaconic, crotonic and vinylphthalic esters and maleic, itaconic and vinylphthalic hemiesters, or unsaturated urethanes selected from acrylic urethane, methacrylic urethane, α-α- dimethyl-m-isopropenylbenzyl urethane, allyl urethane, or allyl ethers, substituted or non-substituted vinyl acrylamide and methacrylamide,

R' represents the hydrophobic radical with a fatty chain formed of linear or branched alkyl groups having 28 carbon atoms,

**d.** optionally, of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of 0.1 $s^{-1}$.

**3.** Partially or totally hydrosoluble copolymer according to the claims 1 or 2, **characterised by** the fact that it is formed:

**a.** of 15% to 98% by weight of an ethylenically unsaturated monomer with a carboxyl function selected from the monoacids, such as acrylic, methacrylic, crotonic, isocrotonic and cinnamic acid, or from itaconic, fumaric, maleic and citraconic acid, carboxylic acid anhydrides, such as maleic anhydride and diacid hemiesters, selected from $C_1$ to $C_4$ monoesters of maleic or itaconic acid,

**b.** of 0% to 83% by weight of an ethylenically unsaturated monomer with no carboxyl function, selected from the group formed by the esters of acrylic or methacrylic acid, such as methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

**c.** of 2% to 18% by weight of an ethylenically unsaturated oxyalkylated monomer terminated by a hydrophobic fatty chain, of general formula (I) according to the claims 1 or 2,

**d.** of 0% to 5% of at least one monomer having at least two ethylenic unsaturations selected from the group

formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of $0.1s^{-1}$.

**4.** Copolymer according to the claims 1 or 2, **characterised by** the fact that it is formed:

**a.** of 20% to 50% by weight of an ethylenically unsaturated monomer with a carboxyl function selected from the monoacids, such as acrylic, methacrylic, crotonic, isocrotonic and cinnamic acid, the diacids such as itaconic, fumaric, maleic and citraconic acid, or from maleic anhydride and diacid hemiesters, selected from $C_1$ to $C_4$ monoesters of maleic or itaconic acid,

**b.** of 47% to 77% by weight of an ethylenically unsaturated monomer with no carboxyl function, selected from the group formed by the esters of acrylic or methacrylic acid, such methyl, ethyl, butyl, 2-ethyl-hexyl acrylates or methacrylates, or by acrylonitrile, vinyl acetate, styrene, methylstyrene, diisobutylene, vinylpyrrolidone, vinylcaprolactam,

**c.** from 3% to 10% by weight of an ethylenically unsaturated oxyalkylated monomer terminated by a hydrophobic fatty chain, of general formula (I) as described in claims 1 or 2,

**d.** of 0% to 3% of at least one monomer having at least two ethylenic unsaturations selected from the group formed by ethyleneglycol dimethacrylate, trimethylolpropanetriacrylate, allyl acrylate, methylene-bis-acrylamide, methylene-bis-methacrylamide, tetrallyloxyethane, triallylcyanurate, allyl ethers obtained from polyols selected from pentaerythritol, sorbitol and sucrose,

and by the fact that the said copolymer, placed in a 0.5% aqueous solution by weight of dry matter brought to a pH of 8.5 by addition of ammonia at 28%, has an apparent viscosity of at least 52 Pa.s measured at 25°C and after 24 hours using a Rheomat 115 viscosimeter equipped with a 145 mobile and for a shear rate of $0.1s^{-1}$.

**5.** Copolymer according to one of claims 1 to 4, **characterised by** the fact that the ethylenically unsaturated monomer with a carboxyl function is selected from the group of acrylic, methacrylic and itaconic acids.

**6.** Copolymer according to one of claims 1 to 4, **characterised by** the fact that the ethylenically unsaturated monomer with no carboxyl function is selected from acrylic esters such as alkyl acrylates and methacrylates having from one to four carbon atoms.

**7.** A process for obtaining a partially or totally hydrosoluble copolymer, cross-linked or not, according to one of claims 1 to 6, which comprises the radical copolymerisation of the monomers to be copolymerised, in solution, in direct or inverted emulsion, in suspension or precipitation in suitable solvents, in the presence of catalytic systems and suitable transfer agents.

**8.** Process for use of the copolymer according to one of claims 1 to 6 as a rheology modifier in cosmetic applications.

**9.** Process for use according to the claim 8 in formulations of the shampoo type.

**10.** Process for use of the copolymer according to one of claims 1 to 6 as a rheology modifier in paste formulations for textile printing.

**11.** Process for use of the copolymer according to one of claims 1 to 6 as an additive to aqueous drilling muds.

**12.** Process for use of the copolymer according to one of claims 1 to 6 as a rheology modifier in detergent applications.

**13.** Process for use according to claim 12 in formulations of the scouring cream type.

**14.** Process for use of the copolymer according to one of claims 1 to 6 as an agent for preventing sedimentation and/or placing in suspension of coarse mineral or organic charges for various applications of the phytosanitary type.

**15.** Process for use of the copolymer according to one of claims 1 to 6 as a rheology modifier in paint formulations.

**Patentansprüche**

**Patentansprüche für folgende Vertragsstaaten : BE, CH, DE, DK, GB, IT, LI, NL, SE**

**1.** Teilweise oder vollständig wasserlösliches, vemetztes oder unvernetztes Copolymer, **dadurch gekennzeichnet, dass** es die folgenden Bestandteile aufweist:

a. mindestens ein ethylenisch ungesättigtes Monomer mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, Anhydriden von Carbonsäuren wie Maleinsäureanhydrid und Halbestern zweiwertiger Säuren wie den Monoestern mit 1 bis 4 C-Atomen der Malein- oder Itaconsäure,

b. gegebenenfalls mindestens ein ethylenisch ungesättigtes Monomer ohne Carboxyl-Funktion, ausgewählt aus der Gruppe bestehend aus Estern, Amiden und/oder Nitrilen der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. mindestens ein oxyalkyliertes, ethylenisch ungesättigtes Monomer, endend mit einer hydrophoben Fettkette, der allgemeinen Formel (I):

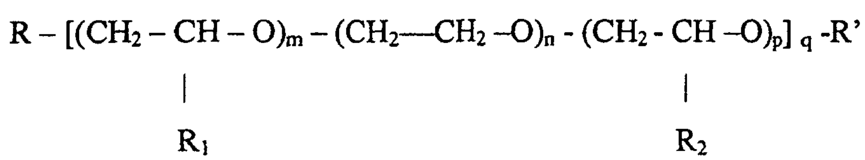

in der:

- m und p eine Anzahl an Alkylenoxidgruppen kleiner oder gleich 100 darstellen,
- n eine Anzahl von Ethylenoxidgruppen kleiner oder gleich 100 darstellt,
- q eine Anzahl von mindestens 1 darstellt, und z.B.:

$$0 < q(n+m+p) \leq 100$$

- $R_1$ Wasserstoff oder einen Methylrest darstellt,
- $R_2$ Wasserstoff oder einen Methylrest darstellt.

R einen ungesättigten, polymerisierbaren Rest darstellt, der zur Gruppe der Acryl-, Methacryl-, Maleinsäure-, Itakonsäure-, Crotonsäure-, Vinylphthalsäureester gehört, sowie Halbester der Maleinsäure, Itakonsäure, Vinylphthalsäure oder auch ungesättigte Urethane, ausgewählt aus Acrylurethan, Methacrylurethan, α-α-Dimethyl-m-isopropenylbenzylurethan, Allylurethan, oder auch Allylether, substituiertes oder nicht substituiertes Acrylamid und Methacrylamid, Vinyle.

R' einen hydrophoben Rest mit Fettkette darstellt, gebildet aus geradkettigen oder verzweigten Alkyl-, Alkylaryl, Arylalkyl-, Arylgruppen mit 26 bis einschließlich 30 Kohlenstoffatomen.

d. gegebenenfalls mindestens ein Monomer mit mindestens zwei ungesättigten Ethylengruppen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethem, erhalten aus

Polyalkoholen, ausgewählt aus Pentaerythritol, Sorbitol, Saccharose.

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**2.** Teilweise oder vollständig wasserlösliches Copolymer nach Anspruch 1, **dadurch gekennzeichnet, dass** es die folgenden Bestandteile aufweist:

a. mindestens ein ethylenisch ungesättigtes Monomer mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, Anhydriden von Carbonsäuren wie Maleinsäureanhydrid und Halbestern zweiwertiger Säuren wie den Monoestem mit 1 bis 4 C-Atomen der Malein- oder Itaconsäure,

b. gegebenenfalls mindestens ein ethylenisch ungesättigtes Monomer ohne Carboxyl-Funktion, ausgewählt aus der Gruppe bestehend aus Estern, Amiden und/oder Nitrilen der Acryl- oder Methacrylsäure wie Methyl-, Ethyl-, Butyl-, 2-Ethylhexylacrylat oder methacrylat oder aus Acrylnitril, Vinylacetat, Styrol, Methylstyrol, Diisobutylen, Vinylpyrrolidon, Vinylcaprolactam,

c. mindestens ein oxyalkyliertes, ethylenisch ungesättigtes Monomer, endend mit einer hydrophoben Fettkette, der allgemeinen Formel (I):

$$R-[(CH_2-\underset{\displaystyle R_1}{\underset{|}{CH}}-O)_m-(CH_2-CH_2-O)_n-(CH_2-\underset{\displaystyle R_2}{\underset{|}{CH}}-O)_p]_q-R'$$

in der:

- m und p eine Anzahl an Alkylenoxidgruppen kleiner oder gleich 100 darstellen,
- n eine Anzahl von Ethylenoxidgruppen kleiner oder gleich 100 darstellt,
- q eine Anzahl von mindestens 1 darstellt, und z.B.:

$$0 < q(n+m+p) \leq 100$$

- $R_1$ Wasserstoff oder einen Methylrest darstellt,
- $R_2$ Wasserstoff oder einen Methylrest darstellt;

R einen ungesättigten, polymerisierbaren Rest darstellt, der zur Gruppe der Acryl-, Methacryl-, Maleinsäure-, Itakonsäure-, Crotonsäure-, Vinylphthalsäureester gehört, sowie Halbester der Maleinsäure, Itakonsäure, Vinylphthalsäure oder auch ungesättigte Urethane, ausgewählt aus Acrylurethan, Methacrylurethan, $\alpha$-$\alpha$-Dimethyl-m-isopropenylbenzylurethan, Allylurethan, oder auch Allylether, substituiertes oder nicht substituiertes Acrylamid und Methacrylamid, Vinyle.

R' einen hydrophoben Rest mit Fettkette darstellt, bestehend aus geradkettigen oder verzweigten Alkylgruppen mit 28 Kohlenstoffatomen.

d. gegebenenfalls mindestens ein Monomer mit mindestens zwei ungesättigten Ethylengruppen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, erhalten aus Polyalkoholen, ausgewählt aus Pentaerythritol, Sorbitol, Saccharose.

und dass das genannte Copolymer, in eine wässrige Lösung mit 0,5 Gew.-% Trockensubstanz gebracht, die mittels

Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**3.** Teilweise oder vollständig wasserlösliches Copolymer nach den Ansprüchen 1 oder 2, **dadurch gekennzeichnet, dass** es die folgenden Bestandteile aufweist:

a. 15 bis 98 Gew.-% eines ethylenisch ungesättigten Monomers mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, Anhydriden von Carbonsäuren wie Maleinsäureanhydrid und Halbestern zweiwertiger Säuren, ausgewählt aus Monoestern mit 1 bis 4 C-Atomen der Malein- oder Itaconsäure,

b. 0 bis 83 Gew.-% eines ethylenisch ungesättigten Monomers ohne Carboxyl-Funktion, ausgewählt aus der Gruppe bestehend aus Estern der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. 2 bis 18 Gew.-% eines oxyalkylierten, ethylenisch ungesättigten Monomers, endend mit einer hydrophoben Fettkette, der allgemeinen Formel (I) nach den Ansprüchen 1 oder 2.

d. 0 bis 5 % mindestens eines Monomers mit mindestens zwei ethylenischen Nichtsättigungen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, erhalten aus Polyalkoholen, ausgewählt aus Pentaerythritol, Sorbitol, Saccharose.

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**4.** Copolymer nach den Ansprüchen 1 oder 2, **dadurch gekennzeichnet, dass** es die folgenden Bestandteile aufweist:

a. 20 bis 50 Gew.-% eines ethylenisch ungesättigten Monomers mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, den Anhydriden von Carbonsäuren wie Maleinsäureanhydrid und Halbestern zweiwertiger Säuren wie den Monoestern mit 1 bis 4 C-Atomen der Malein- oder Itaconsäure,

b. 47 bis 77 Gew.-% eines ethylenisch ungesättigten Monomers ohne Carboxyl-Funktion, ausgewählt aus der Gruppe bestehend aus Estern der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. 3 bis 10 Gew.-% eines oxyalkylierten, ethylenisch ungesättigten Monomers, endend mit einer hydrophoben Fettkette, der allgemeinen Formel (I) nach den Ansprüchen 1 oder 2;

d. 0 bis 3 % mindestens eines Monomers mit mindestens zwei ethylenischen Nichtsättigungen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, die aus Polyalkoholen, ausgewählt aus Pentaerythritol, Sorbitol, Saccharose, erhalten wurden;

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wird, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**5.** Copolymer nach einem der Ansprüche 1 bis 4, **dadurch gekennzeichnet, dass** das ethylenisch ungesättigte Monomer mit Carboxyl-Funktion ausgewählt wird aus der Gruppe bestehend aus Acryl-, Methacryl- und Itaconsäure.

**6.** Copolymer nach einem der Ansprüche 1 bis 4, **dadurch gekennzeichnet, dass** das ethylenisch ungesättigte Monomer ohne Carboxyl-Funktion ausgewählt wird aus Acrylestern wie den Alkylacrylaten und -methacrylaten mit einem bis vier Kohlenstoffatomen.

**7.** Verwendung des Copolymers nach einem der Ansprüche 1 bis 6 als Rheologie-Modifikationsmittel in kosmetischen Anwendungen.

**8.** Verwendung nach Anspruch 7 in Formulierungen vom Shampoo-Typ.

**9.** Verwendung des Copolymers nach einem der Ansprüche 1 bis 6 als Rheologie-Modifikationsmittel in Textildruckmassen-Formulierungen.

**10.** Verwendung des Copolymers nach einem der Ansprüche 1 bis 6 als Additiv für wässerige Bohrschlämme.

**11.** Verwendung des Copolymers nach einem der Ansprüche 1 bis 6 als Rheologie-Modifikationsmittel in Detergensanwendungen.

**12.** Verwendung nach Anspruch 11 in Formulierungen vom Typ Scheuerpaste.

**13.** Verwendung des Copolymers nach einem der Ansprüche 1 bis 6 als Antisedimentations-Mittel und/oder Mittel, um grobe mineralische oder organische Füllstoffe für unterschiedliche Anwendungen im Bereich Pflanzenschutz zu suspendieren.

**14.** Verwendung des Copolymers nach einem der Ansprüche 1 bis 6 als Rheologie-Modifikationsmittel in Farbformulierungen.

**Patentansprüche für folgenden Vertragsstaat : ES**

**1.** Teilweise oder vollständig wasserlösliches, vernetztes oder unvernetztes Copolymer, **dadurch gekennzeichnet, dass** es die folgenden Bestandteile aufweist:

a. mindestens ein ethylenisch ungesättigtes Monomer mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, Anhydriden von Carbonsäuren wie Maleinsäureanhydrid und Halbestern zweiwertiger Säuren wie den Monoestern mit 1 bis 4 C-Atomen der Malein- oder Itaconsäure,

b. gegebenenfalls mindestens ein ethylenisch ungesättigtes Monomer ohne Carboxyl-Funktion, ausgewählt aus der Gruppe bestehend aus Estem, Amiden und/oder Nitrilen der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. mindestens ein oxyalkyliertes, ethylenisch ungesättigtes Monomer, endend mit einer hydrophoben Fettkette, der allgemeinen Formel (I):

$$R-[(CH_2-\underset{\underset{R_1}{|}}{CH}-O)_m-(CH_2-CH_2-O)_n-(CH_2-\underset{\underset{R_2}{|}}{CH}-O)_p]_q-R'$$

in der:

- m und p eine Anzahl an Alkylenoxidgruppen kleiner oder gleich 100 darstellen,

- n eine Anzahl von Ethylenoxidgruppen kleiner oder gleich 100 darstellt,
- q eine Anzahl von mindestens 1 darstellt, und z.B.:

$$0 < q(n+m+p) \leq 100$$

- $R_1$ Wasserstoff oder einen Methylrest darstellt,
- $R_2$ Wasserstoff oder einen Methylrest darstellt.

R einen ungesättigten, polymerisierbaren Rest darstellt, der zur Gruppe der Acryl-, Methacryl-, Maleinsäure-, Itakonsäure-, Crotonsäure-, Vinylphthalsäureester gehört, sowie Halbester der Maleinsäure, Itakonsäure, Vinylphthalsäure oder auch ungesättigte Urethane, ausgewählt aus Acrylurethan, Methacrylurethan, $\alpha$-$\alpha$-Dimethyl-m-isopropenylbenzylurethan, Allylurethan, oder auch Allylether, substituiertes oder nicht substituiertes Acrylamid und Methacrylamid, Vinyle.

R' einen hydrophoben Rest mit Fettkette darstellt, gebildet aus geradkettigen oder verzweigten Alkyl-, Alkylaryl, Arylalkyl-, Arylgruppen mit 26 bis einschließlich 30 Kohlenstoffatomen.

d. gegebenenfalls mindestens ein Monomer mit mindestens zwei ungesättigten Ethylengruppen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, erhalten aus Polyalkoholen, ausgewählt aus Pentaerythritol, Sorbitol, Saccharose,

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**2.** Teilweise oder vollständig wasserlösliches Copolymer nach Anspruch 1, **dadurch gekennzeichnet, dass** es die folgenden Bestandteile aufweist:

a. mindestens ein ethylenisch ungesättigtes Monomer mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, Anhydriden von Carbonsäuren wie Maleinsäureanhydrid und Halbestern zweiwertiger Säuren wie den Monoestem mit 1 bis 4 C-Atomen der Malein- oder Itaconsäure,

b. gegebenenfalls mindestens ein ethylenisch ungesättigtes Monomer ohne Carboxyl-Funktion, ausgewählt aus der Gruppe bestehend aus Estern, Amiden und/oder Nitrilen der Acryl- oder Methacrylsäure wie Methyl-, Ethyl-, Butyl-, 2-Ethylhexylacrylat oder methacrylat oder aus Acrylnitril, Vinylacetat, Styrol, Methylstyrol, Diisobutylen, Vinylpyrrolidon, Vinylcaprolactam,

c. mindestens ein oxyalkyliertes, ethylenisch ungesättigtes Monomer, endend mit einer hydrophoben Fettkette, der allgemeinen Formel (I):

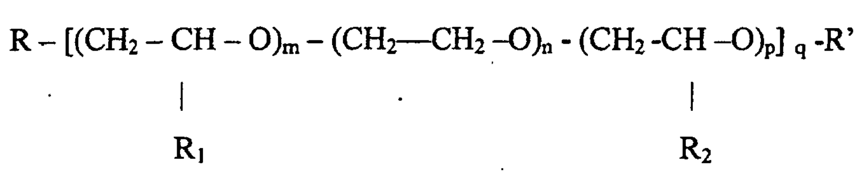

in der:

- m und p eine Anzahl an Alkylenoxidgruppen kleiner oder gleich 100 darstellen,
- n eine Anzahl von Ethylenoxidgruppen kleiner oder gleich 100 darstellt,
- q eine Anzahl von mindestens 1 darstellt, und z.B.:

$$0 < q(n+m+p) \leq 100$$

- $R_1$ Wasserstoff oder einen Methylrest darstellt,
- $R_2$ Wasserstoff oder einen Methylrest darstellt;

R einen ungesättigten, polymerisierbaren Rest darstellt, der zur Gruppe der Acryl-, Methacryl-, Maleinsäure-, Itakonsäure-, Crotonsäure-, Vinylphthalsäureester gehört, sowie Halbester der Maleinsäure, Itakonsäure, Vinylphthalsäure oder auch ungesättigte Urethane, ausgewählt aus Acrylurethan, Methacrylurethan, $\alpha$-$\alpha$-Dimethyl-m-isopropenylbenzylurethan, Allylurethan, oder auch Allylether, substituiertes oder nicht substituiertes Acrylamid und Methacrylamid, Vinyle.

R' einen hydrophoben Rest mit Fettkette darstellt, bestehend aus geradkettigen oder verzweigten Alkylgruppen mit 28 Kohlenstoffatomen.

d. gegebenenfalls mindestens ein Monomer mit mindestens zwei ungesättigten Ethylengruppen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, erhalten aus Polyalkoholen, ausgewählt aus Pentaerythritol, Sorbitol, Saccharose,

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**3.** Teilweise oder vollständig wasserlösliches Copolymer nach den Ansprüchen 1 oder 2, **dadurch gekennzeichnet, dass** es die folgenden Bestandteile aufweist:

a. 15 bis 98 Gew.-% eines ethylenisch ungesättigten Monomers mit Carboxyl-Funktion, ausgewählt aus Monosäuren wie Acryl-, Methacryl-, Croton-, Isocroton-, Zimtsäure, oder auch aus Itacon-, Fumar-, Malein-, Zitrakonsäure, Anhydriden von Carbonsäuren wie Maleinsäureanhydrid und Halbestern zweiwertiger Säuren, ausgewählt aus Monoestern mit 1 bis 4 C-Atomen der Malein- oder Itaconsäure,

b. 0 bis 83 Gew.-% eines ethylenisch ungesättigten Monomers ohne Carboxyl-Funktion, ausgewählt aus der Gruppe bestehend aus Estern der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. 2 bis 18 Gew.-% eines oxyalkylierten, ethylenisch ungesättigten Monomers, endend mit einer hydrophoben Fettkette, der allgemeinen Formel (I) nach den Ansprüchen 1 oder 2.

d. 0 bis 5 % mindestens eines Monomers mit mindestens zwei ethylenischen Nichtsättigungen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, erhalten aus Polyalkoholen, ausgewählt aus Pentaerythritol, Sorbitol, Saccharose,

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**4.** Copolymer nach den Ansprüchen 1 oder 2, **dadurch gekennzeichnet, dass** es die folgenden Bestandteile aufweist:

a. 20 bis 50 Gew.-% eines ethylenisch ungesättigten Monomers mit Carboxyl-Funktion, ausgewählt aus Monosäuren wie Acryl-, Methacryl-, Croton-, Isocroton-, Zimtsäure, zweiwertigen Säuren wie Itacon-, Fumar-, Malein-, Zitrakonsäure, oder aus Maleinsäureanhydrid und Halbestern zweiwertiger Säuren, ausgewählt aus den Monoestern mit 1 bis 4 C-Atomen der Malein- oder Itaconsäure,

b. 47 bis 77 Gew.-% eines ethylenisch ungesättigten Monomers ohne Carboxyl-Funktion, ausgewählt aus der Gruppe bestehend aus Estern der Acryl- oder Methacrylsäure wie Methyl-, Ethyl-, Butyl-, 2-Ethylhexylacrylat oder -methacrylat oder aus Acrylnitril, Vinylacetat, Styrol, Methylstyrol, Diisobutylen, Vinylpyrrolidon, Vinylcaprolactam,

c. 3 bis 10 Gew.-% eines oxyalkylierten, ethylenisch ungesättigten Monomers, endend mit einer hydrophoben Fettkette, der allgemeinen Formel (I) nach den Ansprüchen 1 oder 2;

d. 0 bis 3 % mindestens eines Monomers mit mindestens zwei ethylenischen Nichtsättigungen, ausgewählt aus der Gruppe bestehend aus Ethylenglycoldimethacrylat, Trimethylolpropantriacrylat, Allylacrylat, Methylenbis-acrylamid, Methylen-bis-methacrylamid, Tetraallyloxyethan, Triallylcyanurat, Allylethern, die aus Polyalkoholen, ausgewählt aus Pentaerythritol, Sorbitol, Saccharose, erhalten wurden;

und dass das genannte Copolymer, in eine wässrige Lösung mit 0,5 Gew.-% Trockensubstanz gebracht, die mittels Zugabe von 28 %igem Ammoniak auf einen pH von 8,5 gebracht wurde, eine scheinbare Viskosität von mindestens 52 Pa.s aufweist, gemessen bei 25 °C und nach 24 Std. mit Hilfe des Viskosimeters Rheomat 115, ausgestattet mit einem Mobilteil 145, bei einer Schergeschwindigkeit von 0,1 $s^{-1}$.

**5.** Copolymer nach einem der Ansprüche 1 bis 4, **dadurch gekennzeichnet, dass** das ethylenisch ungesättigte Monomer mit Carboxyl-Funktion ausgewählt wird aus der Gruppe, bestehend aus Acryl-, Methacryl- und Itaconsäure.

**6.** Copolymer nach einem der Ansprüche 1 bis 4, **dadurch gekennzeichnet, dass** das ethylenisch ungesättigte Monomer ohne Carboxyl-Funktion ausgewählt wird aus Acrylestern wie den Alkylacrylaten und -methacrylaten mit einem bis vier Kohlenstoffatomen.

**7.** Verfahren zur Herstellung eines teilweise oder vollständig wasserlöslichen, vernetzten oder unvemetzten Copolymers nach einem der Ansprüche 1 bis 6, das die radikalische Copolymerisation der zu copolymerisierenden Monomere in Lösung, in direkter oder inverser Emulsion, in Suspension oder Fällung in geeigneten Lösungsmitteln in Gegenwart von katalytischen Systemen und geeigneten Übertragungsmitteln umfasst.

**8.** Verfahren zur Verwendung des Copolymers nach einem der Ansprüche 1 bis 6 als Rheologie-Modifikationsmittel in kosmetischen Anwendungen.

**9.** Verfahren zur Verwendung nach Anspruch 8 in Formulierungen vom Shampoo-Typ.

**10.** Verfahren zur Verwendung des Copolymers nach einem der Ansprüche 1 bis 6 als Rheologie-Modifikationsmittel in Textildruckmassen-Formulierungen.

**11.** Verfahren zur Verwendung des Copolymers nach einem der Ansprüche 1 bis 6 als Additiv für wässerige Bohrschlämme.

**12.** Verfahren zur Verwendung des Copolymers nach einem der Ansprüche 1 bis 6 als Rheologie-Modifikationsmittel in Detergensanwendungen.

**13.** Verfahren zur Verwendung nach Anspruch 12 in Formulierungen vom Typ Scheuerpaste.

**14.** Verfahren zur Verwendung des Copolymers nach einem der Ansprüche 1 bis 6 als Antisedimentationsmittel und/oder Mittel, um grobe mineralische oder organische Füllstoffe für unterschiedliche Anwendungen im Bereich Pflanzenschutz zu suspendieren.

**15.** Verfahren zur Verwendung des Copolymers nach einem der Ansprüche 1 bis 6 als Rheologie-Modifikationsmittel in Farbformulierungen.